# EUROPÄISCHE PATENTSCHRIFT

(11) **EP 1 791 517 B1**
(45) Veröffentlichungstag und Bekanntmachung des Hinweises auf die Patenterteilung: **10.08.2011**
(21) Anmeldenummer: 05789519.5
(22) Anmeldetag: 31.08.2005
(51) Int. Cl.: A61K 8/06, A61Q 1/04, A61Q 1/06, A61Q 1/02, A61Q 1/08, A61Q 19/00, A61Q 1/10, A61Q 17/04

(54) **ZUBEREITUNG, INSBESONDERE KOSMETISCHE ZUBEREITUNG, VERFAHREN ZU IHRER HERSTELLUNG UND IHRE VERWENDUNG**
PREPARATION, PARTICULARLY A COSMETIC PREPARATION, METHOD FOR THE PRODUCTION AND USE THEREOF
PREPARATION, EN PARTICULIER PREPARATION COSMETIQUE, SON PROCEDE DE PRODUCTION ET SON UTILISATION

(30) Priorität: 06.09.2004 DE 202004014004 U; 20.09.2004 DE 202004014644 U; 11.11.2004 DE 202004017760 U; 03.05.2005 DE 102005020583
(43) Veröffentlichungstag der Anmeldung: 06.06.2007
(73) Patentinhaber: Schwan-STABILO Cosmetics GmbH & Co. KG, 90562 Heroldsberg (DE)
(72) Erfinder: EMIG, Susanne, 90427 Nürnberg (DE); ENGELHARDT, Sonja, 90562 Heroldsberg (DE); HARGRAVE, Sabine, Sacramento, CA 95831 (US)
(74) Vertreter: Leissler-Gerstl, Gabriele
(86) Internationale Anmeldenummer: PCT/EP2005/009388
(87) Internationale Veröffentlichungsnummer: WO 2006/027151

(56) Entgegenhaltungen:
- EP-A- 1 543 811
- WO-A1-96/18374
- US-A- 5 763 479
- US-A1- 2003 133 895
- US-A1- 2003 185 772
- US-A1- 2003 206 883
- US-A1- 2004 120 913
- US-A1- 2005 008 596
- US-B1- 6 607 717

## Beschreibung

Die Erfindung betrifft eine Zubereitung in Form einer Emulsion mit einer lipophilen äußeren Phase und einer hydrophilen inneren Phase, insbesondere eine kosmetische Zubereitung, sowie deren Herstellung und Verwendung.

Emulsionsartige Zubereitungen, werden auf vielen Gebieten verwendet, insbesondere im Bereich der Kosmetik und der Lebensmittelchemie. Emulsionen sind heterogene Systeme aus zwei Phasen, die nicht oder nicht unbegrenzt miteinander mischbar sind, wobei eine innere Phase in kleinteiliger Form in der anderen äußeren Phase verteilt ist. Die äußere Phase, die die innere Phase in Form feiner bis feinster Tröpfchen enthält, wird als kontinuierliche Phase bezeichnet und bestimmt den Grundcharakter der Emulsion. Emulsionen, in denen Wasser die innere Phase bildet, haben vorteilhafte Eigenschaften. Unter anderem treten mikrobiologische Probleme bei dieser Art von Emulsionen eher nicht auf.

Als äußere Phase werden für solche Emulsionen z. B. Lipide, wie Öle oder Fette, oder Silikone verwendet. Das Silikon kann flüchtig oder nicht flüchtig sein und es kann sich um ein Silikonöl, Silikonharz, Silikongel oder um Silikon-Copolymere handeln.

Um das Emulsionssystem über längere Zeit stabil zu halten und zu verhindern, dass die Tröpfchen der inneren Phase zusammenfließen und sich als Phase abscheiden, ist es notwendig, Emulgatoren zuzusetzen, die einen hydrophilen und einen hydrophoben Anteil aufweisen und sich um die Tröpfchen lagern.

Auf dem Gebiet der Kosmetik häufig verwendete Emulsionen mit äußerer Ölphase, so genannte W/O-Emulsionen, haben oft den Nachteil, dass die äußere lipophile Phase nach dem Auftragen auf der Haut ein schmieriges oder klebriges Gefühl zurücklässt, das als unangenehm empfunden wird. Auch bei der Verwendung von Silikonen als äußerer Phase kann diese unangenehme Wirkung auftreten. Bei Verwendung solcher Emulsionen im Bereich der Augen tritt darüber hinaus der Nachteil auf, dass die Öl- oder Silikonphase in das Auge oder den Bindehautsack wandert und zu einem öligen Schleier auf der Linse und unangenehmen Reizungen führt.

Nachteilig bei den bekannten Zubereitungen ist, dass sie sich von der Haut oder auch den Lippen, auf die sie bestimmungsgemäß aufgebracht wurden, leicht auf andere Oberflächen übertragen, z.B. auf Tassen, Gläser, Textilien oder auch andere Hautbezirke. Dies kann Spuren in Form eines farbigen Abdrucks oder eines Fettfilms hinterlassen. Solche Produkte weisen also eine ungenügende Haftung auf dem Untergrund auf, was dazu führt, dass Lippen- und Wangenrouge, Make-up, Lidschatten und auch Sonnenschutzmittel regelmäßig neu aufgetragen werden müssen. Da ölige Bestandteile meist sehr gut auf der Haut und den Lippen spreiten, wandern die Pigmente vom ursprünglichen Auftragsort zusammen mit geringen Mengen aus der Ölphase in die Feinfältelung

der Haut der unmittelbaren Umgebung, was oft den optischen Gesamteindruck sehr störend und negativ beeinflusst.

In der Vergangenheit versuchte man dem bei Lippenstiften und Lippenrouge durch den Einsatz sog. "bromo acids", Farbstoffen, die substantiv auf die Haut aufziehen, zu begegnen. Da diese Farbstoffe aber aufgrund unterschiedlicher pH-Werte der Haut individuell verschiedene und nicht vorhersagbare Tönungen gaben und zudem die Einfärbungen oft tagelang anhielten, wurde dieser Weg alsbald wieder verlassen, weil die Verbraucherinnen derartige Produkte nur ungenügend akzeptierten.

Nachdem Silikonöle und Silikonharze Eingang in die Kosmetik gefunden hatten, wurde versucht die Haftung auf der Haut und damit die Haltbarkeit von dekorativen Zubereitungen hierdurch zu verbessern. So sind seit etwa 1977 Lidschatten- und Lippenstifte in Form von Minen bekannt, die in spitzbare Umhüllungen eingegossen wurden, welche in der Lipidphase unter anderem eine Mischung aus Phenyltrimethicone (ein nicht flüchtiges Silikonöl) und Cyclomethicone (ein flüchtiges Silikonöl) enthielten. Ihnen folgten dann gleichartige Zubereitungen nach, die Cyclomethicone als alleinige Silikonkomponente enthielten. Diese Stiftmassen ließen sich - obwohl sie eine scheinbar feste Struktur aufwiesen - weich und geschmeidig, ähnlich einer pastösen Masse, auf die Haut auftragen. Derartige Massen werden in zahlreichen Anmeldungen beschrieben; beispielsweise in DE 27 18 957, DE 27 59 610, DE 27 59 856, DE 30 28 231 und können mit den dort beschriebenen Verfahren hergestellt werden. DE 40 05 894 beschreibt darüber hinaus eine Methode, um derartige Stiftmassen abzufüllen.

Nach dem Abdampfen des flüchtigen Silikons verblieb ein weicher, elastischer Film auf der Haut, der sehr gut haftete und nur minimal in die Umgebung des ursprünglichen Auftragsortes auswanderte: Das Prinzip der Kombination zweier Silikonöle, übertragen auf pastöse Zubereitungen, findet sich beispielsweise auch wieder in EP 0 756 864.

Obwohl Silikonöle in diesen Zubereitungen der bisher bekannten Art Haftung und Haltbarkeit verbessern konnten, bewirkten die Silikonöle bei empfindlichen Anwendern nachteilige Effekte, wenn sie in unmittelbarer Augennähe angewendet wurden. Gelangen nämlich selbst minimale Mengen von Silikonölen, insbesondere nicht flüchtigen Silikonölen oder sonstigen Silikonpolymeren aus den aus dem Stand der Technik bekannten, stiftförmigen Produkten in das Auge oder den Bindehautsack, so können sie zu einem öligen Schleier auf der Linse und zu unangenehmen Reizungen, einem sog. "wind burn effect" führen.

Ein weiteres Problem bietet sich im Hinblick auf Emulsionen, die teilchenförmige oder feinteilige Inhaltsstoffe, wie Pigmente oder Effektstoffe enthalten. Die Teilchen können die Emulsion destabilisieren und sich absetzen, sodass eine ästhetisch unbefriedigende zweiphasige Zusammensetzung entsteht.

Aufgabe der Erfindung war es daher, eine Zubereitung, insbesondere eine kosmetische Zubereitung zu schaffen, die die Nachteile der Produkte des Standes der Technik nicht aufweist, und die als geschmeidige Paste vorliegt. Eine weitere Aufgabe war es, eine Zubereitung zur Verfügung zu stellen, die über längere Zeit stabil bleibt und bei der sich die Phasen nicht trennen, auch wenn teilchenförmige Inhaltsstoffe enthalten sind.

Aufgabe der Erfindung war es weiterhin, eine lagerstabile Zusammensetzung bereitzustellen, die sich auch bei längerer Lagerung bei unterschiedlichen Temperaturen in unterschiedlichen Klimazonen nicht in Phasen auftrennt. Außerdem soll sich die Zubereitung gut auftragen lassen und am aufgetragenen Ort haften und nach dem Trocknen weder in die Umgebung wandern noch sich auf andere Oberflächen übertragen.

Eine weitere Aufgabe der Erfindung war es, eine Zubereitung zur Verfügung zu stellen, die frei von vom Tier abgeleiteten Stoffen herstellbar ist, d.h. die ausschließlich Inhaltsstoffe aus Pflanzen und/oder mineralische und/oder synthetische Materialien enthält.

Weiterhin war es eine Aufgabe der Erfindung, eine Zubereitung bereitzustellen, die Effektmaterialien gut zur Geltung bringt und glänzen lässt, ohne einen störenden "Fettglanz" zu erzeugen.

Diese Aufgaben werden mit einer Zubereitung, wie sie in Anspruch 1 definiert ist, gelöst.

Die erfindungsgemäße Zubereitung liegt in Form einer Emulsion mit einer lipophilen äußeren Phase und einer hydrophilen inneren Phase vor, wobei die äußere Phase eine mindestens zweiwertige Esterkomponente, ein flüchtiges Silikon, einen Emulgator, eine teilchenförmige Phase und gegebenenfalls ein Feuchthaltemittel und weitere in der Kosmetik übliche Inhaltstoffe enthält, und die innere Phase ein wässriges Medium und ggf. in der Kosmetik übliche hydrophile Zusatzstoffe enthält. wobei die Esterkomponente einen Schmelzpunkt im Bereich von 40 bis 200°C hat.

Überraschenderweise wurde gefunden, dass eine Zubereitung, wie sie beansprucht wird, die Nachteile des Standes der Technik nicht zeigt. Insbesondere bleibt sie über längere. Zeit stabil, haftet ausgezeichnet am aufgetragenen Ort, ist wischfest und überträgt sich praktisch nicht auf andere Oberflächen, wie Tassen, Gläser, Textilien oder auch andere Hautbezirke. Sie hinterlässt auch keine Spuren in Form eines farbigen Abdrucks oder eines Fettfilms. Da sie keine öligen Bestandteile enthält, die auf der Haut und den Lippen spreiten, wandern die Pigmente nicht vom Auftragsort weg in die Feinfältelung der Haut. Es werden daher mit der erfindungsgemäßen Zubereitung ausgezeichnete optische Ergebnisse erzielt. Die Zubereitung hat eine ausgeprägte Strukturviskosität und lässt sich daher leicht und geschmeidig und in unterschiedlicher Schichtdicke auftragen, so dass nach dem Auftrag sanfte und weiche Übergänge zur unbehandelten Haut gemacht werden können. Im Gegensatz zu bekannten silikonhaltigen Zusammensetzungen lässt sie die Haut nicht austrocknen und erzeugt auch kein spannendes Gefühl. Die erfindungsgemäße Zubereitung wandert nach dem Trocknen weder auf der Haut noch in die Lippenfältchen. Trotz des Gehalts an Silikonen spannt sie nicht und

trocknet die Haut nicht aus. Die Lagerstabilität bei den unterschiedlichen Temperaturen der unterschiedlichen Klimazonen ist ausgezeichnet und erfüllt damit gesetzliche Anforderungen. Auch nach längerer Lagerzeit zeigt sie keinerlei Synäreseeffekte.

Die erfindungsgemäße Zubereitung liegt als Emulsion in Form einer geschmeidigen Paste vor und eignet sich für kosmetische Anwendungen, insbesondere im Bereich der dekorativen Kosmetik, zum Färben und Verschönen der Haut, der Lippen und der Augenlider. Beispielhaft genannt seien hier Lippen- und Wangenrouge, Make-up, Concealer, Lidschatten, Mittel zur Fixierung von Lippenstift oder Lippenrouge, Pftegegrundlage zur Pflege der Haut oder Sonnenschutzmittel.

Die vorteilhaften, Eigenschaften werden durch Kombination ausgewählter Inhaltsstoffe erzielt. Die in Anspruch 1 aufgeführten und im Folgenden näher erläuterten Inhaltsstoffe tragen die guten Eigenschaften bei. Durch Verwendung dieser Inhaltsstoffe kann auf die Verwendung von fettigen oder öligen Bestandteilen, insbesondere solchen, die sich vom Tier ableiten, verzichtet werden. Außerdem wird der Anteil an nichtflüchtigen Silikonen so stark verringert, dass deren nachteilige Eigenschaften nicht zum Tragen kommen.

Es wurde gefunden, dass bei einer Zubereitung, insbesondere einer kosmetischen Zubereitung in Form einer Emulsion, wie sie in Anspruch 1 definiert ist, die Kombination aus flüchtigem Silikonöl, Esterkomponente, Teilchenphase und Wasser keine weiteren Ölkomponenten erforderlich macht, um ein stabiles guthaftendes produkt zu erhalten. Auf niedrigviskose nicht flüchtige Silikonöle kann im wesentlichen verzichtet werden; sie werden, wenn überhaupt nur in unschädlichen ganz geringen Mengen unterhalb von 5 Gew.-% eingesetzt, z.B. um die Trocknungszeit der Zubereitung zu verzögern und gleichzeitig als Entschäumer und Emulsionshilfe.

Auf fettige und ölige Komponenten und nachteilige Silikonverbindungen zu verzichten, wird ermöglicht, indem erfindungsgemäß eine Kombination aus flüchtigen Silikonen mit speziellen Estern verwendet wird. Diese Kombination gewirkt, dass sich die Viskosität auch während einer längeren Lagerzeit nur unwesentlich verändert.

Die erfindungsgemäße Zubereitung in Form einer Emulsion weist eine innere und eine äußere Phase auf. Die innere oder disperse Phase ist wässrig und besteht aus einem wässrigen Medium, in dem die hydrophilen Bestandteile dispergiert und/oder ggf. gelöst sind Die äußere Phase enthält wenigstens eine mehrwertige Esterkomponente, wenigstens ein flüchtiges Silikon und wenigstens eine teilchenförmige Phase. Die Zubereitung enthält darüber hinaus noch mindestens einen Emulgator und kann zusätzlich gegebenenfalls noch weitere in der Kosmetik übliche Zusatzstoffe enthalten.

Die wesentlichen Bestandteile der erfindungsgemäßen Zubereitung sorgen dafür, dass eine stabile Emulsion entsteht. Dazu enthält die erfindungsgemäße Zubereitung eine wachsartige Esterkomponente in Kombination mit einem flüchtigen Silikon, die für die Struktur sorgen, mindestens einen Emulgator, der die Emulsion stabilisiert und ein wässriges Medium als dispergierte Phase. Darüber hinaus sind vorteilhaft wenigstens ein Feuchthaltemittel und wenigstens eine feinteilige Feststoffphase enthalten.

Das wässrige Medium besteht aus Wasser und/oder wässrigen oder mit Wasser mischbaren Lösungsmitteln. Das wässrige Medium kann z.B. eine Wasser-Alkohol-Mischung sein, wobei der Alkohol bevorzugt Ethanol oder Isopropanol ist. Bevorzugt ist das wässrige Medium Wasser.

Die äußere Phase enthält als erfindungswesentlichen Bestandteil eine mehrwertige Esterkomponente, die zur Strukturbildung beiträgt und als wachsartiger Bestandteil eingesetzt wird. Als mehrwertig werden im Rahmen der vorliegenden Erfindung mindestens zweiwertige Ester verstanden. Dabei werden als mehrwertige Esterkomponenten solche Verbindungen definiert, die aus mehrwertigen (mindestens zweiwertigen) Alkoholen mit jeweils mindestens zwei Säureresten, mehrwertigen (mindestens zweiwertigen) Säuren und jeweils mindestens zwei Alkoholresten oder mehrwertigen Alkoholen und mehrwertigen Säuren aufgebaut sind, wobei die Kettenlänge der vom Alkohol stammenden Reste und der von der Säure stammenden Reste C₂ bis C₆₀, bevorzugt C₄ bis C₆₀, besonders bevorzugt C₂₀ bis C₆₀ ist.

Die Esterkomponente soll in der Zubereitung unter anderem die Funktion eines Wachses übernehmen und hat daher bevorzugt einen Schmelzpunkt im Bereich zwischen 40 und 200°C, bevorzugt von 55 bis 125°C.

Um eine Esterkomponente mit dem angegebenen Schmelzpunkt zu erhalten, sollte die Summe der Kohlehstoffatome aus Alkohol- und Carbonsäureresten in einem Bereich von 35 bis 150, bevorzugt 40 bis 100 liegen. Sowohl Alkoholrest als auch Carbonsäurerest können einen gesättigten oder ein- oder mehrfach ungesättigten geradkettigen oder verzweigtkettigen Kohlenwasserstoffanteil aufweisen und ggf. auch noch weitere Substituenten in Form von funktionellen Gruppen, wie Hydroxyl-, Carboxyl-, Amino-, Säureamid-, Estergruppen und dergl. aufweisen.

Bevorzugt enthält die mehrwertige Esterkomponente mindestens eine Verbindung der Formel I: 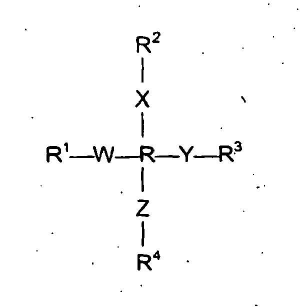 worin R ein linearer, verzweigter oder cyclischer Kohlenwasserstoffrest mit 1 bis 8 Kohlenstoffatomen ist, W, X, Y und Z jeweils unabhängig voneinander -C(O)O-, -OC(O)-, -O-, -NR⁵₂ oder -NC(O)- ist, und jeder der Reste R¹, R², R³, R⁴ und R⁵ jeweils unabhängig einen linearen oder verzweigten langkettigen Kohlenwasserstoff, bevorzugt mit 8 bis 36, besonders bevorzugt 12 bis 24 Kohlenstoffatomen, bedeutet.

Besonders gute Eigenschaften werden erzielt, wenn Ester, insbesondere Fettsäureester des Pentaerythrits, wie z.B. Pentaterythrityl-Tetramyristat, Tristearat, Tetrastearat, Triisostearat, Tetraisostearat, Tri-(12-hydroxy)-stearat, Tetra-(12-hydroxy)-stearat, Tribehenat, Tetrabehenat, Tetra-(ethylhexyldodecanoat), Trierucat, Tetraerucat, Tetramelissinat und ähnliche verwendet werden.

Zusätzlich zu der Esterkomponente kann noch mindestens ein pflanzliches Wachs, bevorzugt ein Gemisch aus pflanzlichen Wachsen, insbesondere ein Gemisch aus Candelillawachs und Carnaubawachs verwendet werden. Gemische aus der erfindungsgemäßen Esterkomponente und Wachsen sind möglich unter der Prämisse, dass unerwünschte Wechselwirkungen zwischen den Einzelkomponenten minimiert werden sollten.

Die Esterkomponenten wird in einer Menge eingesetzt, die zur Erreichung der gewünschten "Nullviskosität" entsprechend dem gewünschten Einsatzzweck erforderlich ist. Die Nullviskosität und komplexe Viskosität kann mit einem Rheometer des Typs MCR-301 von Anton-Paar (Messsystem Platte/Platte, Platten beide sandgestrahlt, Plattendurchmesser 25 mm, Spaltbreite 1.000 µm; Scherrate bei der Nullviskosität 0,00005 s⁻¹; Temperatur 298,15 K; Software Rheoplus/32 V6.23) bestimmt werden.

Die Einsatzmenge liegt dabei in einem Mengenbereich zwischen 0,5 und 20 Gew.-%, bevorzugt in einem Mengenbereich zwischen 2 und 12 Gew.-%. Überraschend wurde festgestellt, dass die erfindungsgemäß verwendeten Esterkomponenten, insbesondere die von Pentaerythrit abgeleiteten Ester - möglicherweise aufgrund der sich vom Tetraedermodell ableitenden dreidimensionalen Raumstruktur - sehr gut geeignet sind, flüchtige Silikonöle, wie z.B. Cyclomethicone oder kurzkettige Dimethylpolysiloxane zu gelieren und auf diese Art Strukturen auszubilden, die das Aufbringen der erfindungsgemäßen Zubereitung erleichtern.

Falls zusätzlich ein Wachs der erfindungsgemäßen Zubereitung zugefügt wird, ist es bevorzugt ein Wachs mit einem Tropfpunkt zwischen 50 und 200 °C, insbesondere zwischen 60 und 150 °C, ganz besonders bevorzugt zwischen 75 und 120 °C. Diese zusätzliche Wachskomponente enthält wenigstens einen Alkoholrest einer Kettenlänge zwischen C₂ und C₆₀ und einen Carbonsäurerest mit einer Kettenlänge zwischen C₄ und C₆₀. Sowohl Alkoholrest als auch Carbonsäurerest können einen gesättigten oder ein- oder mehrfach ungesättigten geradkettigen oder verzweigtkettigen Kohlenwasserstoffanteil aufweisen und ggf. auch noch weitere Substituenten in Form von funktionellen Gruppen, wie Hydroxyl-, Carboxyl-, Amino-, Säureamid-, Estergruppen und dergl. aufweisen. Das Wachs wird, falls verwendet, in einem solchen Anteil eingesetzt, dass die gewünschte Nullviskositäf erreicht wird bzw. erhalten bleibt.

Der zweite wesentliche Bestandteil der erfindungsgemäßen Zubereitung ist ein flüchtiges Silikonöl. Unter flüchtigem Silikönöl wird hier eine Silikonverbindung verstanden, die bei Raumtemperatur-flüssig ist und dann, wenn sie auf die Haut aufgetragen wird, d.h. bei einer Temperatur zwischen 30 und 38°C, langsam verdampft. Geeigneterweise hat das flüchtige Silikon einen Dampfdruck im Bereich von 0,1 bis 20 kPa bei 25°C, bevorzugt 0,2 bis 10 kPa und besonders bevorzugt 0,5 bis 5 kPa.

Erfindungsgemäß kann eine Zubereitung, insbesondere kosmetische Zubereitung in Form einer Emulsion bereitgestellt werden, die als einzige flüssige Lipidkomponente flüchtige Silikonöle enthält. Geeignet sind kurzkettige und cyclische Dimethylsiloxane. Beispiele hierfür sind Cyclomethicone wie Hexamethylcyclotrisiloxan, Octamethylcyclotetrasiloxan, Decamethylcyclopentasiloxan, Dodecamethylcyclohexasiloxan oder kurzkettige Dimethicone wie Hexamethyldisiloxan, Octamethyltrisiloxan, Decamethyltetrasiloxan, Dodecamethylpentasiloxan oder Mischungen daraus, wobei deren Einsatzmenge zwischen 5 und 65 Gew.-%, bezogen auf das Gesamtgewicht der Zubereitung, bevorzugt zwischen 15 und 45 Gew.-% liegt,

In der Zubereitung können ggf. nicht flüchtige Silikonöle in geringer Menge, z.B. als Entschäumer und Emulgierungshilfsmittel, vorhanden sein. Die Menge der nicht flüchtigen Silikonöle liegt unterhalb von 5 Gew.-%, bevorzugt unter 1,5 Gew.%.

Die Nachteile von aus dem Stand der Technik bekannten Zubereitungen, die Öle und Fette enthalten, können erfindungsgemäß überwunden werden, indem als flüssige hydrophobe Komponenten nur flüchtiges Silikonöl und gegebenenfalls eine geringe Menge nichtflüchtiger Silikonöle, die als Entschäumer und Emulgierungshilfsmittel eingesetzt werden, verwendet werden. Es wurde festgestellt, dass durch die erfindungsgemäße Kombination von Esterkomponente und flüchtigem Silikonöl eine sehr gute Konsistenz erzielt wird, ohne die Nachteile, die Lipidkomponenten schaffen.

Die erfindungsgemäße Kombination ermöglicht es, dass in der Zubereitung eine feste Phase stabil integriert wird, welche z.B. aus feinteiligen Füllstoffen, Färbemitteln und/oder Effektstoffen bestehen kann.

Die vorgenannte feste oder teilchenförmige Phase besteht aus Inhaltstoffen in Teilchenform, die sich weder in der hydrophoben noch in der hydrophilen Phase lösen. Die teilchenförmige Phase kann z.B. aus Füllstoffen, wie z.B. Talkum, Kaolin, Stärke und modifizierte Stärke, Polytetrafluorethylenpulver (Teflon), Nylonpulver, Bornitrid, aus unlöslichen Metallseifen, wie Mg-Stearat, Ca-Stearat, Sr-Stearat, Zn-Stearat, aus Effektmaterialien, wie Flitter, Glitter, fluoreszierenden und phosphoreszierenden Teilchen, und insbesondere aus anorganischen oder organischen Pigmenten oder aus Mischungen der genannten Stoffe bestehen. Als Pigmente seien beispielhaft genannt: Titandioxid, Zinkoxid, Eisenoxide, Chromoxid, Chromoxidhydrat, Ultramarin, Berliner Blau (Ferric Blue), Glimmer, Perlglanzmittel wie z.B. mit Titandioxid beschichtete Glimmer, farbige; mit Titandioxid und Metalloxiden beschichtete Glimmer, Bismuthoxidchlorid, beschichtetes Bismuthoxidchlorid, plättchenförmige Metallpulver von Aluminium, Messing, Bronze, Kupfer, Silber, Gold, sowie Verlackungen organischer Färbemittel mit Aluminium, Barium, Zirkonium, Calcium oder Strontium. Diese Aufzählung ist nur beispielhaft und nicht abschließend.

Im Falle von Sonnenschutzmitteln können besonders feinteilige Pigmente, sog. Nanopigmente mit einer durchschnittlichen Teilchengröße zwischen 5 und 50 nm, Anwendung finden, welche auf der Haut transparent wirken und sie nicht mehr einfärben. Beispielhaft genannt seien hier Siliciumdioxid, Titandioxid, Ceroxid, Aluminiumoxid, Zirkonoxid und Zinkoxid.

In einer bevorzugten Ausführungsform werden als Färbemittel Verlackungen organischer Farbstoffe eingesetzt. Es wurde gefunden, dass die Kombination aus Verlackungen organischer Farbstoffe mit der erfindungsgemäßen Emulsion bewirkt, dass die Farbstoffe nicht ausbluten, sondern in der erzeugten Struktur erhalten bleiben.

Diese Zusätze erfolgen mit der Maßgabe, dass sie von der jeweiligen nationalen oder regionalen Kosmetik-Gesetzgebung auch zugelassen sind. Auch die Einsatzmengen liegen bevorzugt im Rahmen der durch die jeweilige Kosmetik-Gesetzgebung erlaubten Höchstmengen. Die Mengenanteile an Feststoffen, insbesondere an Pigmenten, liegen dabei, soweit keine gesetzlichen Regelungen dem entgegenstehen, in einem Bereich von 0 bis 40 Gew.-%, vorzugsweise in einem Bereich von 5 bis 30 Ges.-% und ganz besonders bevorzugt in einem Bereich von 8 bis 20 Gew.-%.

In einer weiteren bevorzugten Ausführungsform wird eine Zubereitung bereitgestellt, die Perlglanzpigmente und blättchenförmige Metallpulver enthält. Überraschenderweise wurde festgestellt, dass bei erfindungsgemäßen Zubereitungen, wenn sie Färbemittel auf Basis von Perlglanzpigmenten und plättchenförmigen Metallpulvern enthalten, ein ungewöhnlich intensiver Glanz entsteht, der, ohne an eine Theorie gebunden zu sein, darauf zurückgeführt wird, dass sich in der erfindungsgemäßen Emulsion die Pigmentteilchen, vielleicht bedingt durch das gesteuerte Abdunsten der flüchtigen Bestandteile Silikonöl und Wasser, ausrichten und damit die Möglichkeit entsteht, dass sie sich an aktive Ladungszentren der Haut ausrichten.

Zur Bildung der erfindungsgemäßen Emulsionen wird wenigstens ein Emulgator verwendet. Es kann auch eine Mischung von Emulgatoren eingesetzt werden. Bevorzugt wird ein für W/S-Emulsionen geeigneter Emulgator oder eine kombination aus einem W/S- und einem W/O-Emulgator verwendet. Es können die dem Fachmann bekannten Emulgatoren zugegeben werden. Bevorzugt wird ein nichtionogener Emulgator verwendet. Bevorzugte Emulgatoren sind solche, die beispielsweise ausgewählt sind unter Sorbitan-Sesquioleat, Sorbitan-Laurat, Polyglyceryl-4 Isostearat, PEG-5 Soya Sterol, Soya Sterol, Polyglyceryl-2-PEG-4 Isostearat, Polyglyceryl-2 Sesquiisostearat oder Cetyl PEG/PPG Dimethicone, wie z.B. Cetyl PEG/PPG-10/1 Dimethicone. Pflanzliche Sterole, wie Soyasterole eignen sich dabei erfahrungsgemäß bevorzugt als Hilfsemulgatoren. Grundsätzlich können natürlich auch Gemische aus den vorgenannten Emulgatoren verwendet werden - unter der Prämisse, dass unerwünschte Wechselwirkungen zwischen den Einzelkomponenten minimiert werden sollen, wird jedoch bevorzugt nur einer der vorgenannten Emulgatoren zur Herstellung der erfindungsgemäßen Emulsion eingesetzt. Grundsätzlich geeignet sind aber auch Phosphatester als Emulgatoren zur Herstellung der erfindungsgemäßen Zubereitung, wie z.B. Trioleyl-Phosphat, Trioleth-8-Phosphat oder Trilaureth-4-Phosphat.

Die erforderlichen Einsatzmengen an Emulgatoren liegen im Bereich zwischen 0,5 und 10 Gew.-%, bevorzugt zwischen 1,5 und 6 Gew.-%. Zur Stabilisierung der erfindungsgemäßen Emulsionen können der Wasserphase ggf. in Wasser leicht lösliche anorganische Salze oder (in Wasser nahezu unlösliche) Salze von Fettsäuren zugesetzt werden, wie z.B. Magnesiumsulfat, Natriumsulfat, Natriumchlorid, Kaliumchlorid, Magnesiumstearat oder Magnesiummyristat. Die beiden letztgenannten sind vor der Emulsionsbildung sorgfältig in der Wasserphase zu dispergieren - sie können bevorzugt aber auch der Feststoffphase zugemischt werden. Die vorgenannten Salze werden in Mengen von 0,05 bis 3 Gew.-%, bevorzugt aber 0,3 bis 2 Gew.% in der Wasserphase eingesetzt.

Zusätzlich können der erfindungsgemäßen Zubereitung, bevorzugt in der wässrigen Phase, noch wenigstens ein Feuchthaltemittel wie z.B. Propylenglykol, Dipropylenglykol, Tripropylenglykol, Butylenglykol, Glycerin, Diglycerin, Triglycerin, Sorbitol, Mannitol, Xylitol, Glucose, Fructose, Sucrose, Carbamid (Harnstoff), Milchsäure, Zitronensäure, Pyrrolidoncarbonsäure (PCA) oder die Salze der genannten Säuren oder Mischungen aus den genannten Stoffen zugesetzt werden. Bevorzugt werden hierbei die in Wasser leicht löslichen Natriumsalze eingesetzt. Die Einsatzmengen betragen 0,1 bis 5 Gew.-%, bevorzugt 1 bis 3 Gew.-%.

Zur Stabilisierung über einen breiteren Temperaturbereich können Silica (amorphe Kieselsäure), Bentonite, Hectorite, Montmorillonite oder dgl. eingesetzt werden.

Weiterhin kann die erfindungsgemäße Zubereitung in der Kosmetik übliche Zusätze wie Konservierungsmittel, Antioxidantien, Riechstoffe, Vitamine, Sonnenschutzfilter und dgl. in den für diese Stoffe üblichen Mengen enthalten.

Die erfindungsgemäße Zubereitung zeichnet sich dadurch aus, dass sich die Viskosität auch während einer längeren Lagerzeit nur unwesentlich verändert. Ein besonderer Vorteil der erfindungsgemäßen Zubereitung besteht darin, dass sich auch spezifisch schwerere Inhaltsstoffe, z.B. teilchenförmige Materialien wie Pigmente und Effektstoffe nach längerer Zeit nicht absetzen.

Ein weiterer Vorteil der Erfindung besteht darin, dass eine Zubereitung, insbesondere eine kosmetische Zubereitung zur Verfügung gestellt werden kann, die ausschließlich von Pflanzen ableitbare und/oder mineralische und/oder synthetische Inhaltsstoffe enthält, jedoch völlig frei ist von Stoffen, die sich vom Tier ableiten.

Um gegenseitige Wechselwirkungen zwischen den eingesetzten Inhaltsstoffen weitestgehend zu minimieren, soll die Basis dieser Zubereitung - ohne die ggf. eingesetzte Feststoffphase - zudem aus einer möglichst geringen Zahl von Stoffen zusammengesetzt sein.

Die erfindungsgemäße Zubereitung weist vorteilhafte viskoelastische Eigenschaften auf, die sich in den Werten für die komplexe Viskosität und die Nullviskosität wiederspiegeln. So können erfindungsgemäß Zusammensetzungen erhalten werden, deren komplexe Viskosität im Bereich von 800 bis 6.000 Pa·s liegt und deren Nullviskosität zwischen 200.000 und 1.200.000 Pa·s liegt (Scherrate bei der Nullviskosität 0,00005 s⁻¹Temperatur 298,15 K; Messsystem Platte/Platte, Platten beide sandgestrahlt, Plattendurchmesser 25 mm, Spaltbreite 1.000 µm; Messgerät MCR-301; Anton-Paar; Software Rheoplus/32 V6.23).

Die vorgenannten erfindungsgemäßen Zubereiturigen können wegen ihrer ausgezeichneten Haltbarkeit und Deckkraft in Verbindung mit den überaus guten Applikationseigenschaften durchaus auch als Camouflage zum Kaschieren von Altersflecken oder Rosacea, Concealer und dgl., ferner Sonnenschutzprodukte mit unterschiedlichen Lichtschutzfaktoren (SPF), bis hin zu sog. Sunblockern unter Verwendung von sehr feinteiligen Nanopigmenten oder bunt eingefärbten, höher pigmentierten Sunblockern, welche als Körperbemalung bei Surfern und Windsurfern beliebt sind, Verwendung finden. Die genannten Nanopigmente liegen vorzugsweise in einer Teilchengröße von 5 bis 50 nm vor und können ausgewählt sein unter Titandioxid, Zinkoxid, Ceroxid oder Aluminiumoxid. Sie werden vorzugsweise eingesetzt in Mengen von 2 bis 20 Gew.%, besonders bevorzugt in Mengen von 5 bis 10 Gew.-%.

Die Zubereitung kann auch ohne Zusatz von Färbemitteln hergestellt werden und ggf. sog. kosmetische Wirkstoffe enthalten. Sie findet dann Verwendung als

Fixierungsmittel, welches über einen Lippenstift oder einem Lippenrouge aufgetragen wird. Enthält diese uneingefärbte Zübereitung Lichtschutzfilter, so kann sie als Lippenschutz und -pflege verwendet werden. Die Haut der Lippen enthält ja bekanntermaßen, im Gegensatz zur Haut des Körpers, keine Pigmentierung. Geeignete öllösliche Lichtfiltersubstanzen, die im Bereich des UV-A und UV-B guten Schutz bieten, sind dem einschlägig befassten Fachmann in ausreichender Zahl bekannt und durch die jeweilige nationale oder regionale Gesetzgebung z.B. in der EU, in Japan und in den U.S.A. geregelt - in Deutschland beispielsweise durch die Anlage 7 zu § 3b der Kosmetik-Verordnung und sollen hier deshalb nicht umfassend aufgelistet werden. Beispielhaft erwähnt seien deshalb nur das Isoamyl p-Methoxycinnamate als UV-B Filter und 4-Methylbenzylidene Camphor als UV-A Filter.

Die erfindungsgemäße Zubereitung liegt vor in Form einer weichen geschmeidigen Paste, welche sich leicht und gleichmäßig applizieren und verteilen läßt. Aufgrund ihres Wassergehaltes in der inneren (dispersen) Phase erzeugt sie beim Auftragen auf die Haut, in Verbindung mit den flüchtigen Silikonölen, einen angenehm kühlenden Effekt. Sie kann in den Anwendern bekannter Weise wieder von der Haut entfernt werden - durch geeignete Abschminkmittel oder -tücher oder durch Waschen mit Feinseife oder geeigneten milden Tensidzubereitungen. Sie kann in bekannter Weise in geeignete Behältnisse, wie Flaschen, ggf. mit Spatel, Tiegel oder Tuben abgefüllt und daraus vom Anwender wieder entnommen werden. Sie kann aber auch, wegen der damit verbundenen verbesserten hygienischen Verhältnisse, in geeignete Auftragvorrichtungen, sog. Spendermechaniken eingebracht und daraus appliziert werden. Für den Auftrag kleiner Mengen, wie sie z.B. für die Applikation im Lippen- oder Augenbereich benötigt werden, bieten sich Auftragvorrichtungen an, wie sie z.B. aus US 6,238,117 oder aus US 6,309,128 bekannt sind, da diese eine sehr schöne Feindosierung erlauben.

Die erfindungsgemäße Zubereitung kann hergestellt werden, indem die hydrophoben Bestandteile in der hydrophoben Phase gelöst bzw. dispergiert werden, die hydrophilen Bestandteile in der wässrigen Phase gelöst bzw. dispergiert werden, Emulgator einer der beiden Phasen oder beiden zugesetzt wird und dann die Zusammensetzung unter Anwendung von Scherkraft emulgiert wird.

Bevorzugt erfolgt die Herstellung der erfindungsgemäßen Emulsion, indem zuerst die teilchenförmige Phase in dem flüchtigen Silikon dispergiert wird, getrennt davon die Esterkomponente aufgeschmolzen wird und die hydrophoben Inhaltsstoffe zugefügt werden und in einem getrennten Ansatz die wässrige Phase auf die Temperatur der Esterkomponente erwärmt wird und die hydrophilen Inhaltsstoffe zugefügt werden. Anschließend werden alle drei Ansätze zusammengeführt, homogenisiert und anschließend gekühlt, wobei bevorzugt die Emulsion bewegt wird. Sobald die Emulsion etwa Körpertemperatur erreicht hat, können die temperaturempfindlichen Bestandteile, wie Duftstoffe zugefügt Werden. Zuletzt wird die .fertige Zubereitung verpackt.

Die erfindungsgemäße Zubereitung, insbesondere kosmetische Zubereitung soll anhand der nachfolgenden Beispiele im Detail erläutert werden, welche sie jedoch nicht abschließend beschreiben. Dabei erfolgen alle Mengenangaben in Gewichtsprozent (Gew.-%), bezogen jeweils auf : das Gesamtgewicht der Zubereitung, die Bezeichnung der Rohstoffe erfolgt mit den dem einschlägig befaßten Fachmann allgemein bekannten "INCI-Namen:

| **Beispiel 1 -Creme-Lidschatten, kühlend (non-transfer)** | |
|---|---|
| Decamethylcycloperitasiloxane | 26,300 |
| Pentaerythrityl Tetrabehenate | 5,900 |
| Cetyl PEG/PPG-10/1 Dimethicone | 3,800 |
| Glycerin | 3,750 |
| Sodium Chloride | 0,800 |
| Methylparaben | 0,200 |
| Propylparaben | 0,100 |
| Phenoxyethanol | 0,500 |
| Ascorbyl Palmitate | 0,100 |
| Tocopherol | 0,350 |
| Fragrance | 0.300 |
| Titanated Mica (C.I.-No 77019, 77891) | 9,500 |
| Titanium Dioxide (C.I.-No. 77891) | 3,500 |
| Red Iron Oxide (C.I.-No. 77491) | 1,000 |
| Yellow Iron Oxide (C.I.-No. 77492) | 0,600 |
| Black Iron Oxide (C.I.-No. 77499) | 0,400 |
| Aqua | 42,900 |

Die Herstellung erfolgt, indem man die Pigmente mit dem Silikonöl sehr intensiv in einer geeigneten Homogenisiermaschine mit Ankerrühnrverk und Zahnkranzhomogenisator vorlegt und mittels Homogenisator intensiv dispergiert Danach wird das Tetrabehenyl Tetrabehenate zusammen mit dem Emulgator zugegeben und bei etwa 70-80°C aufgeschmolzen. Man setzt nun der Schmelze die Parabene, Phenoxyethanol, das Ascorbyl Palmitate und das Tocopherol zu. In einem separaten Gefäß wurden das Glycerin und das Sodium Chloride im Wasser gelöst und dann die Wasserphase auf etwa 70°C erhitzt. Die Wasserphase wird nun unter gutem Rühren in die Homogenisiermaschine eingesaugt. Nach Ende der Zugabe wird der Ansatz intensiv homogenisiert und dann unter gutem Rühren abgekühlt. Bei etwa 45°C wird nochmals kurz homogenisiert, dann unter Rühren weiter abgekühlt auf etwa 38°C. Bei dieser Temperatur wird Fragrance (das Riechstoffgemisch) zugegeben und nochmals kurz homogenisiert. Der Ansatz wird nun durch Anlegen von maximalem Vakuum entlüftet und bis zum Erreichen von etwa 25°C weitergerührt. Danach wird ausgetragen und in geeignete Lagergebinde abgefüllt. Man erhält hierbei eine Zubereitung in Form einer hellbraunen, stark glänzenden, wasserfesten, geschmeidigen Paste mit einer Nullviskosität von 853.000 Pas.

| **Beispiel 2 - Cremelidschatten, kühlend (non-transfer)** | |
|---|---|
| Hexamethyldisiloxane | 25,500 |
| Pentaerythrityl-Tetrabehenate | 4,600 |
| Sorbitan Sesquioleate | 3,800 |
| 1.3-Butandiol | 3,500 |
| Titanated Mica (C.I. 77019, 77891) | 15,500 |
| Ultramarine Blue (C.I. 77013) | 5,800 |
| Chromium Hydroxide Green (C.I. 77289) | 1,200 |
| Black Iron Oxide (C.I. 77499) | 0,800 |
| Tocopherol | 0,500 |
| Fragrance | 0,150 |
| Methylparaben | 0,200 |
| Propylparaben | 0,150 |
| Ascorbyl Palmitate | 0,100 |
| Magnesium Sulfate | 0,700 |
| Aqua | 37,500 |

Die Herstellung erfolgt analog zu der in Beispiel 1 beschriebenen Verfahrensweise. Naturgemäß ist darauf zu achten, dass das Pearlpigment, wie dem einschlägig befassten Fachmann geläufig, nicht zu lange starken Scherkräften ausgesetzt wird. Man erhält eine grünstichig -blaue, wasserfeste, geschmeidige Paste mit ausgeprägtem Kühleffekt, die sich gut auf die Augenlider auftragen lässt. Die obige Zubereitung weist eine Nullviskosität von 451.000 Pas auf. Erfahrungsgemäß können sich bei der Verwendung anderer Pigmentkombinationen in anderen Mengen auch etwas andere Viskositätswerte ergeben.

| | |
|---|---|
| **Beispiel 3 - Lippenrouge (kühlend, wasserfest)** | |
| Hexamethyldisiloxane | 23,500 |
| Pentaerythrityl Tetraerucate | 5,200 |
| Trioleyl Phosphate | 4,100 |
| 1.3-Butylenglykol | 3,500 |
| Magnesium Sulfate | 1,000 |
| Methylparaben | 0,200 |
| Propylparaben | 0,100 |
| Phenoxyethanol | 0,500 |
| Ascorbyl Palmitate | 0,100 |
| Tocopherol | 0,300 |
| Fragrance | 0,150 |
| Titanated Mica (C.I.-No. 77019, 77891) | 1,500 |
| Titanium Dioxide (C.I.-No. 77891) | 2,800 |
| Red Iron Oxide (C.I.-No. 77491) | 2,200 |
| FD&C Yellow No. 5 AI-Lake (C.I. 19140:1) | 0,800 |
| FD&C Red No. 3 AI-Lake (C.I. 45430:1) | 0,700 |
| Aqua | 43,350 |

Die Herstellung erfolgt analog der vorstehend beschriebenen Verfahrensweise. Man erhält eine stark glänzende, kräftig rote Zubereitung in Form einer wasserfesten, geschmeidigen Paste mit einer Nullviskosität von 491.000 Pas, die sich besonders gut für die weiter oben beschriebenen automatischen Auftragvorrichtungen eignet..

| **Beispiel 4 -. Lippenrouge mit Lichtschutzfilter (kühlend, wasserfest)** | |
|---|---|
| Decamethylcyclopentasiloxane | 22,500 |
| Dodecamethylcyclohexasiloxane | 3,500 |
| Pentaerythrityl Tetraerucate | 4,800 |
| Polyglyceryl-2-PEG-4 lsostearate | 3,800 |
| Glycerin | 3,700 |
| Sodium Chloride | 0,600 |
| Methylparaben | 0,200 |
| Propylparaben | 0,150 |
| Phenoxyethanol | 0,600 |
| Ascorbyl Palmitate | 0,100 |
| Tocopherol | 0,250 |
| Fragrance | 0,150 |
| Titanated Mica (C.I. 77019, 77891) | 7,000 |
| Titanium Dioxide Nanopigment (C.I. 77891) | 6,000 |
| Isoamyl p-Methoxycinnamate | 1,500 |
| 4-Methylbenzylidene Camphor | 2,000 |
| Red Iron Oxide (C.I. 77491) | 2,000 |
| Titanium Dioxide (C.I. 77891) | 2,500 |
| D&C Red No. 6, Ba-Lake (C.I. 15850:2) | 3,500 |
| Aqua | 35,150 |

Die Herstellung erfolgt analog zu den vorstehend beschriebenen Beispielen. Man erhält eine kräftig rot gefärbte, geschmeidige Paste mit feinem Perlglanz, zum Schutz der Lippen vor starker Sonneneinstrahlung. Sie weist eine Nullviskosität im Bereich von 386.000 Pas auf.

| **Beispiel 5 - Sunblocker für Surfer (wasserfest)** | |
|---|---|
| Hexamethyldisiloxane | 24,000 |
| Phenyltrimethicone | 0,800 |
| Pentaerythrityl Tetrabehenate | 4,800 |
| Pentaerythrityl Tetra-(12-hydroxy)-stearate | 1,000 |
| Sorbitan Sesquioleate | 4,200 |
| lron Oxide Red (C.I.-No. 77491) | 3,200 |
| Iron Oxide Yellow C.I.-No. 77492) | 1,800 |
| 1.2-Propandiol | 3,800 |
| Titanium Dioxide (Nanopigment) | 10,000 |
| Iron Oxides (Rot und Gelb) | 5,000 |
| Polyester-3, Orange 5 | 7,500 |
| Isoamyl p-Methoxycinnamate | 3,500 |
| 4-Methylbenzylidene Camphor | 2,500 |
| Tocopherol | 0,600 |
| Fragrance | 0,200 |
| Methylparaben | 0,200 |
| Propylparaben | 0,150 |
| Ascorbyl Palmitate | 0,100 |
| Sodium Chloride | 0,600 |
| Aqua | 26,050 |

Die Herstellung erfolgt analog der vorstehend beschriebenen Verfahrensweise. Man erhält eine Zubereitung in Form einer wasserfesten, geschmeidigen Paste mit einer intensiv orangegelben Färbung und einer Nullviskosität von 780.000 Pas.

| **Beispiel 6 - Mittel zur Fixierung von Lippenstift und Lippenrouge (wischfest)** | |
|---|---|
| Decamethylcyclopentasiloxan | 32,500 |
| Phenyltrimethicone | 0,500 |
| Pentaerythrityl Tribehenate | 6,200 |
| Sorbitan Sesquioleate | 4,750 |
| Tocopherol | 0,300 |
| Diglycerin | 3,000 |
| Magnesiumstearat | 2,800 |
| Fragrance | 0,200 |
| Methylparaben | 0,200 |
| Propylparäben | 0,150 |
| Ascorbyl Palmitate | 0,100 |
| Magnesium Sulfate | 0,600 |
| Aqua | 48,700 |

Die Herstellung erfolgt in Analogie zu den vorstehenden Beispielen - hier wir das Magnesiumstearat intensiv im Silikonöl dispergiert - ansonsten wird verfahren, wie in Beispiel 1 beschrieben. Man erhält eine weiße cremeartige Zubereitung, die auf den Lippen einen transparenten Film mit angenehm kühlendem Effekt hinterläßt. Die Zubereitung hat eine Nullviskosität von 685.000 Pas.

## Patentansprüche

1. Zubereitung in Form einer Emulsion mit einer lipophilen äußeren Phase und einer hydrophilen inneren Phase, wobei die äußere Phase mindestens einen mehrwertigen Ester mindestens ein flüchtiges Silikon und gegebenenfalls weitere in der Kosmetik übliche lipophile Zusatzstoffe enthält und die innere Phase ein wässriges Medium und ggf. in der Kosmetik übliche hydrophile Zusatzstoffe enthält, wobei die Zubereitung weiterhin mindestens einen Emulgator und eine Feststoffphase enthält, wobei der mehrwertige Ester aus mindestens zweiwertigen Alkoholen mit jeweils mindestens zwei Säureresten, oder mindestens zweiwertigen Säuren und jeweils mindestens zwei Alkoholresten oder mehrwertigen Alkoholen und mehrwertigen Säuren aufgebaut ist, wobei die Kettenlänge der vom Alkohol stammenden Reste C₂ bis C₆₀ ist, und die Kettenlänge der von Säuren stammenden Reste C₄ bis C₆₀ ist , wobei der Ester einen Schmelzpunkt im Bereich von 40 bis 200°C hat.

2. Zubereitung, insbesondere kosmetische Zubereitung nach Anspruch 1, **dadurch gekennzeichnet, dass** der mehrwertige Ester weitere funktionelle Gruppen ausgewählt aus Hydroxyl-, Carboxyl-, Amino-, Säureamid- oder Estergruppen aufweist.

3. Zubereitung nach Anspruch 1 oder 2, **dadurch gekennzeichnet, dass** der mehrwertige Ester eine Verbindung der Formel I: 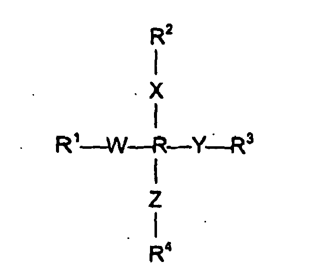 ist, worin R ein linearer, verzweigter oder cyclischer Kohlenwasserstoffrest mit 1 bis 8 Kohlenstoffatomen ist, W, X, Y und Z jeweils unabhängig voneinander -C(O)O-,-OC(O)-, -O-, -NR⁵₂ NC(O)-ist, und jeder der Reste R¹, R², R³, R⁴, und R⁵ jeweils unabhängig einen linearen oder verzweigten langkettigen Kohlenwasserstoffrest bedeutet.

4. Zubereitung nach Anspruch 3, **dadurch gekennzeichnet, dass** der mehrwertige Ester eine Verbindung der Formel I ist, wobei W, X, Y und Z jeweils eine Estergruppe bedeuten, R C bedeutet und mindestens drei der Reste R¹, R², R³, und R⁴ jeweils unabhängig einen C₆ bis C₂₄- Alkylrest bedeuten und der vierte der Reste H oder einen C₆ bis C₂₄- Alkylrest bedeutet.

5. Zubereitung nach einem der vorhergehenden Ansprüche **dadurch gekennzeichnet, dass** in der Esterkomponente die Summe der Kohlenstoffatome aus Alkohol- und Carbonsäureresten in einem Bereich von 35 bis 150 liegt.

6. Zubereitung nach einem der vorstehenden Ansprüche, **dadurch gekennzeichnet, dass** der mehrwertige Ester ein Pentaerythritester ist.

7. Zubereitung nach Anspruch 6, **dadurch gekennzeichnet, dass** der Pentaerythritester Pentaerythrityl-Tetramyristat, Tristearat, Tetrastearat, Trüsostearat, Tetraisostearat, Tribehenat, Tetrabehenat, Tetra-(ethylhexyl-dodecanoat). Tri-(12-hydroxy)-stearat, Tetra-(12-hydroxy)-stearat, Trierucat, Tetraerucat, Tetramelissinat oder eine Mischung davon ist.

8. Zubereitung nach einem der vorhergehenden Ansprüche **dadurch gekennzeichnet, dass** sie ausschließlich von Pflanzen ableitbare und/oder mineralische und/oder synthetische Inhaltsstoffe enthält und dabei völlig frei ist von Stoffen, die sich vom Tier ableiten.

9. Zubereitung nach einem der vorhergehenden Ansprüche, **dadurch gekennzeichnet, dass** sie zusätzlich ein Wachs mit einem Tropfpunkt zwischen 50 und 200 °C, bevorzugt zwischen 60 und 150 °C, ganz besonders bevorzugt zwischen 75 und 120°C aufweist.

10. Zubereitung nach Anspruch 7, **dadurch gekennzeichnet, dass** das Wachs sowohl mindestens einen Alkoholrest als auch mindestens einen Carbonsäurerest aufweist, der einen gesättigten oder ein- oder mehrfach ungesättigten geradkettigen oder verzweigtkettigen Kohlenwasserstoffanteil besitzt.

11. Zubereitung nach Anspruch 10, **dadurch gekennzeichnet, dass** das Wachs ein Gemisch aus Candelillawachs und Carnaubawachs ist.

12. Zubereitung nach einem der vorhergehenden Ansprüche, **dadurch gekennzeichnet, dass** der mehrwertige Ester in einem Bereich zwischen 0,5 und 20 Gew.-%, bevorzugt in einem Bereich zwischen 2 und 12 Gew.-% enthalten ist.

13. Zubereitung nach einem der vorhergehenden Ansprüche, **dadurch gekennzeichnet, dass** das flüchtige Silikonöl ausgewählt ist aus Hexamethylcyclotrisiloxan, Octamethylcyclotetrasiloxan, Decamethylcyclopentasiloxan, Dodecamethylcyclohexasiloxan, Hexamethydisiloxan, Octamethytrisiloxan, Decamethyltetrasiloxan Dodecamethylpentasiloxan oder Gemischen daraus.

14. Zubereitung nach einem der vorhergehenden Ansprüche, **dadurch gekennzeichnet, dass** zusätzlich ein nicht-flüchtiges Silikonöl in einer Menge von weniger als 5 Gew.-% enthalten ist.

15. Zubereitung, insbesondere kosmetische Zubereitung nach Anspruch 14, **dadurch gekennzeichnet, dass** es sich bei dem Silikonöl um ein nicht flüchtiges Silikonöl oder um Mischungen nicht flüchtiger Silikonöle handelt.

16. Zubereitung nach Anspruch 15, **dadurch gekennzeichnet, dass** das nichtflüchtige Silikon ausgewählt ist aus Dimethylpolysiloxanen mit unterschiedlicher Kettenlänge und unterschiedlicher Viskosität oder arylierten Silikonölen, wie Phenyldimethicone, Phenyltrimethicone, Diphenyldimethicone oder Mischungen daraus.

17. Zubereitung nach einem der vorhergehenden Ansprüche, **dadurch gekennzeichnet, dass** der Emulgator ein W/O-Emulgator oder eine Mischung aus W/O-Emulgator und W/S-Emulgator ist.

18. Zubereitung nach Anspruch 17, **dadurch gekennzeichnet, dass** der Emulgator ein nichtionogener W/O-Emulgator ist.

19. Zubereitung nach Anspruch 18, **dadurch gekennzeichnet, dass** der nichtionogene W/O-Emulgator ausgewählt ist aus Sorbitan-Sesquioleat, Sorbitan-Laurat, Soya Sterol, PEG-5 Soya Sterol, Polyglyceryl-4 Isostearat, Polyglyceryl-2-PEG-4 Isostearat, Polyglyceryl-2 Sesquiisostearat, Cetyl-PEG/PPG Dimethicone, Trioleyl-Phosphat, Trioleth-8-Phosphat Trilaureth-4-Phosphat oder Mischungen daraus.

20. Zubereitung nach einem der vorhergehenden Ansprüche, **dadurch gekennzeichnet, dass** ein Stabilisierungsmittel enthalten ist.

21. Zubereitung nach Anspruch 20, **dadurch gekennzeichnet, dass** das Stabilisierungsmittel ein in Wasser leicht lösliches anorganisches Salz ist.

22. Zubereitung nach Anspruch 21, **dadurch gekennzeichnet, dass** das in Wasser leicht lösliche, anorganische Salz ausgewählt ist aus Natriumchlorid, Kaliumchlorid, Natriumsulfat, Magnesiumsulfat oder Mischungen daraus.

23. Zubereitung nach einem der Ansprüche 19 bis 21, **dadurch gekennzeichnet, dass** das Stabilisierungsmittel in einer Menge von 0,05 bis 3 Gew.-%, bevorzugt in einer Menge von 0,3 bis 2 Gew.-% in der Wasserphase enthalten ist.

24. Zubereitung nach einem der vorhergehenden Ansprüche, **dadurch gekennzeichnet, dass** in der Wasserphase zusätzlich ein Feuchthaltemittel enthalten ist.

25. Zubereitung nach Anspruch 24, **dadurch gekennzeichnet, dass** das Feuchthaltemittel ausgewählt ist aus Propylenglykol, Dipropylenglykol, Tripropylenglykol, Butylenglykol, Glycerin, Diglycerin, Triglycerin, Sorbitol, Mannitol, Xylitol, Glucose, Fructose, Sucrose, Carbamid (Harnstoff), Milchsäure, Zitronensäure, Pyrrolidoncarbonsäure (PCA) oder Salzen der genannten Säuren, oder Mischungen der genannten Stoffe enthalten sind.

26. Zubereitung nach einem der Ansprüche 24 oder 25, **dadurch gekennzeichnet, dass** das Feuchthaltemittel in der Wasserphase in einer Menge von 0,1 bis 5 Gew.-%, bevorzugt in einer Menge von 1 bis 3 Gew.-% enthalten ist.

27. Zubereitung nach einem der vorhergehenden Ansprüche, **dadurch gekennzeichnet, dass** die Feststoffphase Füllstoffe, Effektstoffe und/oder anorganische und/oder organische Pigmente oder deren Mischungen aufweist.

28. Zubereitung nach Anspruch 27, **dadurch gekennzeichnet, dass** der Füllstoff ausgewählt ist aus Talkum, Kaolin, Stärke, modifizierter Stärke, Polytetrafluotethylenpulver, Nylonpulver, Bornitrid, Mg-Stearat, Ca-Stearat, Sr-Stearat, Zn-Stearat oder deren Mischungen.

29. Zubereitung nach Anspruch 27 oder 28, **dadurch gekennzeichnet, dass** die Feststoffphase in Mengenanteilen in einem Bereich von 0 bis 40 Gew.-%, bevorzugt 5 bis 30 Gew.-%, besonders bevorzugt 8 bis 20 Gew.-% enthalten ist.

30. Zubereitung nach Anspruch 27, **dadurch gekennzeichnet, dass** das anorganische Pigment ein Nanopigment mit einer Teilchengröße von 5 bis 50 nm ist, ausgewählt aus Titandioxid, Zinkoxid, Zirkonoxid, Ceroxid, Aluminiumoxid, Siliciumdioxid oder Mischungen davon.

31. Zubereitung nach Anspruch 30, **dadurch gekennzeichnet, dass** das Nanopigment in einer Menge von 2 bis 20 Gew.-%, bevorzugt in einer Menge von 5 bis 10 Gew.-% enthalten ist.

32. Zubereitung nach einem der Ansprüche 30 oder 31, **dadurch gekennzeichnet, dass** das Nanopigment mit öllöslichen UV-A- und UV-B-Lichtfiltersubstanzen kombiniert ist.

33. Zubereitung nach Anspruch 32, **dadurch gekennzeichnet, dass** die öllöslichen UV-A- und UV-B-Lichtrftersubstanzen 4-Methylbenzylidene Camphor und Isoamyl p-Methoxycinnamate sind.

34. Zubereitung nach Anspruch 27, **dadurch gekennzeichnet, dass** das anorganische Pigment Titandioxid, Zinkoxid, Eisenoxide, Chromoxid, Chromoxidhydrat, Ultramarin, Berliner Blau (Ferric Blue), Glimmer, mit Titandioxid beschichteter Glimmer, mit Titandioxid und mit Metalloxiden beschichteter Glimmer, Bismuthoxidchlorid, beschichtetes Bismuthoxidchlorid, plätichenförmiges Metallpulver von Aluminium, Messing, Bronze, Kupfer, Silber, Gold oder deren Mischungen ist.

35. Zubereitung nach Anspruch 27, **dadurch gekennzeichnet, dass** das organische Pigment in Form von Verlackungen organischer Färbemittel mit Aluminium, Barium, Calcium, Strontium, Zirkon und von deren Mischungen vorliegt.

36. Zubereitung, insbesondere kosmetische Zubereitung nach einem der vorstehenden Ansprüche, **dadurch gekennzeichnet, dass** sie sich im Bereich der dekorativen Kosmetik zum Pflegen, Färben und Verschönen der Haut, der Lippen und der Augenlider eignet.

37. Zubereitung nach einem der vorhergehenden Ansprüche, **dadurch gekennzeichnet, dass** sie ein Lippenrouge, Wangenrouge, Make-up, Lidschatten, Camouflage oder ein Concealer ist.

38. Zubereitung nach einem der vorhergehenden Ansprüche, **dadurch gekennzeichnet, dass** sie ein Mittel zur Fixierung von Lippenstift oder Lippenrouge, eine Pflegegrundlage, ein Mittel zur Pflege der Haut oder ein Sonnenschutzmittel ist.

39. Zubereitung nach einem der vorstehenden Ansprüche, **dadurch gekennzeichnet, dass** sie eine geschmeidige Paste in Form einer Wasser-in-Silikon-Emulsion mit einer komplexen Viskosität von 800 bis 6.000 Pas und einer Nullviskosität von 200.000 bis 1.200.000 Pas, bevorzugt 400.000 und 900.000 Pas ist (Scherrate bei der Nullviskosität 0.00005 s⁻¹; Temperatur 298,15 K).

40. Zubereitung nach einem der vorhergehenden Ansprüche, **dadurch gekennzeichnet, dass** sie in wiederverschließbare Flaschen, Tiegel oder Tuben abgefüllt ist.

41. Zubereitung nach einem der vorhergehenden Ansprüche, **dadurch gekennzeichnet, dass** sie in eine wiederverschließbare Spendermechanik abgefüllt ist.

42. Verfahren zur Herstellung einer Zubereitung nach einem der Ansprüche 1 bis 41, wobei zuerst die teilchenförmige Phase in dem flüchtigen Silikon dispergiert wird, getrennt davon die Esterkomponente aufgeschmolzen wird und die hydrophoben Inhaltsstoffe zugefügt werden, in einem getrennten Ansatz die wässrige Phase auf die Temperatur der Esterkomponente erwärmt wird und die hydrophilen Inhaltsstoffe zugefügt werden, anschließend alle drei Ansätze zusammengeführt, homogenisiert und anschließend gekühlt werden und ggf. sobald die Emulsion etwa Körpertemperatur erreicht hat, temperaturempfindliche Bestandteile, wie Duftstoffe zugefügt werden.

## Claims

1. A preparation in the form of an emulsion having a lipophilic outer phase and a hydrophilic inner phase, wherein the outer phase contains at least one polyvalent ester, at least one volatile silicone and optionally further lipophilic additives that are usual in cosmetics, and the inner phase contains an aqueous medium and optionally hydrophilic additives that are usual in cosmetics, wherein the preparation further contains at least one emulsifier and a solids phase, wherein the polyvalent ester is built up out of at least divalent alcohols each with at least two acid residues or at least divalent acids and in each case at least two alcohol residues or polyvalent alcohols and polyvalent acids, wherein the chain length of the residues originating from the alcohol is C₂ to C₆₀, and the chain length of the residues originating from acids is C₄ to C₆₀, wherein the ester has a melting point in the range of 40 to 200°C.

2. A preparation, in particular a cosmetic preparation according to claim 1, **characterised in that** the polyvalent ester has further functional groups selected from hydroxyl, carboxyl, amino, acid-amide or ester groups.

3. A preparation according to claim 1 or 2, **characterised in that** the polyvalent ester is a compound of formula I: 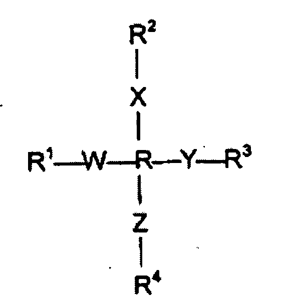 wherein R is a linear, branched or cyclic hydrocarbon residue with 1 to 8 carbon atoms, W, X, Y and Z are each independently of each other -C(O)O-, -OC(O)-, -O-, -NR⁵₂ -NC(O)-, and each of the residues R¹, R², R³, R⁴, and R⁵ respectively independently denotes a linear or branched, long-chain hydrocarbon residue.

4. A preparation according to claim 3, **characterised in that** the polyvalent ester is a compound of formula I, wherein W, X, Y and Z each denote an ester group, R denotes C, and at least three of the residues R¹, R², R³ and R⁹ respectively independently denote a C₆ to C₂₄-alkyl residue, and the fourth of the residues denotes H or a C₆ to C₂₄-alkyl residue.

5. A preparation according to one of the previous claims, **characterised in that** in the ester component the sum of the carbon atoms of alcohol and carboxylic acid residues lies in a range of 35 to 150.

6. A preparation according to one of the preceding claims, **characterised in that** the polyvalent ester is a pentaerythritol ester.

7. A preparation according to claim 6, **characterised in that** the pentaerythritol ester is pentaerythrityl tetramyristate, tristearate, tetrastearate, triisostearate, tetraisostearate, tribehenate, tetrabehenate, tetra-(ethylhexyl-dodecanoate), tri-(12-hydroxy)-stearate, tetra-(12-hydroxy)-stearate, trierucate, tetraerucate, tetramelissinate or a mixture thereof.

8. A preparation according to one of the previous claims, **characterised in that** it contains exclusively ingredients that can be derived from plants and/or are mineral and/or synthetic and in this connection is completely free of substances that are derived from animals.

9. A preparation according to one of the previous claims, **characterised in that** it additionally has a wax with a dropping point between 50 and 200°C, preferably between 60 and 150°C, especially preferably between 75 and 120°C.

10. A preparation according to claim 7, **characterised in that** the wax has both at least one alcohol residue and at least one carboxylic acid residue that has a saturated or singly or multiply unsaturated straight-chain or branched-chain hydrocarbon portion.

11. A preparation according to claim 10, **characterised in that** the wax is a mix of candelilla wax and carnauba wax.

12. A preparation according to one of the previous claims, **characterised in that** the polyvalent ester is contained in a range between 0.5 and 20% by weight, preferably in a range between 2 and 12% by weight.

13. A preparation according to one of the previous claims, **characterised in that** the volatile silicone oil is selected from hexamethyl cyclotrisiloxane, octamethyl cyclotetrasiloxane, decamethyl cyclopentasiloxane, dodecamethyl cyclohexasiloxane, hexamethyl disiloxane, octamethyl trisiloxane, decamethyl tetrasiloxane, dodecamethyl pentasiloxane or mixes thereof.

14. A preparation according to one of the previous claims, **characterised in that** in addition a non-volatile silicone oil is contained in a quantity of less than 5% by weight.

15. A preparation, in particular a cosmetic preparation according to claim 14, **characterised in that** the silicone oil is a non-volatile silicone oil or mixtures of non-volatile silicone oils.

16. A preparation according to claim 15, **characterised in that** the non-volatile silicone is selected from dimethyl polysiloxanes with differing chain length and differing viscosity or arylated silicone oils, such as phenyldimethicone, phenyltrimethicone, diphenyldimethicone or mixtures thereof.

17. A preparation according to one of the previous claims, **characterised in that** the emulsifier is a W/O emulsifier or a mixture of W/O emulsifier and W/S emulsifier.

18. A preparation according to claim 17, **characterised in that** the emulsifier is a non-ionogenic W/O emulsifier.

19. A preparation according to claim 18, **characterised in that** the non-ionogenic W/O emulsifier is selected from sorbitan sesquioleate, sorbitan laurate, soya sterol, PEG-5 soya sterol, polyglyceryl-4 isostearate, polyglyceryl-2-PEG-4 isostearate, polyglyceryl-2 sesquiisostearate, cetyl-PEG/PPG dimethicone, trioleyl-phosphate, trioleth-8-phosphate trilaureth-4-phosphate, or mixtures thereof.

20. A preparation according to one of the previous claims, **characterised in that** a stabilizing agent is contained therein.

21. A preparation according to claim 20, **characterised in that** the stabilizing agent is an inorganic salt that can easily be dissolved in water.

22. A preparation according to claim 21, **characterised in that** the inorganic salt that can easily be dissolved in water is selected from sodium chloride, potassium chloride, sodium sulphate, magnesium sulphate or mixtures thereof.

23. A preparation according to one of claims 19 to 21, **characterised in that** the stabilizing agent is contained in a quantity of 0.05 to 3% by weight, preferably in a quantity of 0.3 to 2% by weight, in the water phase.

24. A preparation according to one of the previous claims, **characterised in that** in addition a moistening agent is contained in the water phase.

25. A preparation according to claim 24, **characterised in that** the moistening agent is selected from propylene glycol, dipropylene glycol, tripropylene glycol, butylene glycol, glycerine, diglycerine, triglycerine, sorbitol, mannitol, xylitol, glucose, fructose, sucrose, carbamide (urea), lactic acid, citric acid, pyrrolidone carboxylic acid (PCA) or salts of said acids or mixtures of said substances.

26. A preparation according to one of claims 24 or 25, **characterised in that** the moistening agent is contained in the water phase in a quantity of 0.1 to 5% by weight, preferably in a quantity of 1 to 3% by weight.

27. A preparation according to one of the previous claims, **characterised in that** the solids phase has fillers, effect substances and/or inorganic and/or organic pigments or mixtures thereof.

28. A preparation according to claim 27, **characterised in that** the filler is selected from talcum, kaolin, starch, modified starch, polytetrafluoroethylene powder, nylon powder, boron nitride, Mg stearate, Ca stearate, Sr stearate, Zn stearate or mixtures thereof.

29. A preparation according to claim 27 or 28, **characterised in that** the solids phase is contained in quantitative proportions in a range of 0 to 40% by weight, preferably 5 to 30% by weight, particularly preferably 8 to 20% by weight.

30. A preparation according to claim 27, **characterised in that** the inorganic pigment is a nano-pigment with a particle size of 5 to 50 nm, selected from titanium dioxide, zinc oxide, zirconium oxide, cerium oxide, aluminium oxide, silicon dioxide or mixtures thereof.

31. A preparation according to claim 30, **characterised in that** the nano-pigment is contained in a quantity of 2 to 20% by weight, preferably in a quantity of 5 to 10% by weight.

32. A preparation according to one of claims 30 or 31, **characterised in that** the nano-pigment is combined with oil-soluble UV-A- and UV-B-light filter substances.

33. A preparation according to claim 32, **characterised in that** the oil-soluble UV-A- and UV-B-light filter substances are 4-methylbenzylidene camphor and isoamyl p-methoxycinnamates.

34. A preparation according to claim 27, **characterised in that** the inorganic pigment is titanium dioxide, zinc oxide, iron oxides, chromium oxide, hydrated chromium oxide, ultramarine, Berlin blue (ferric blue), mica, mica coated with titanium dioxide, mica coated with titanium dioxide and with metal oxides, bismuth oxide chloride, coated bismuth oxide chloride, metal powder in flake form of aluminium, brass, bronze, copper, silver, gold or mixtures thereof.

35. A preparation according to claim 27, **characterised in that** the organic pigment is in the form of lakes of organic colouring agents with aluminium, barium, calcium, strontium, zirconium and mixtures thereof.

36. A preparation, in particular a cosmetic preparation according to one of the preceding claims, **characterised in that** it is suitable in the field of decorative cosmetics for caring for, colouring and enhancing skin, lips and eyelids.

37. A preparation according to one of the previous claims, **characterised in that** it is a lip rouge, blusher, make-up, eye shadow, camouflage or a concealer.

38. A preparation according to one of the previous claims, **characterised in that** it is an agent for fixing lipstick or lip rouge, a care foundation, an agent for caring for the skin or a sunscreen agent.

39. A preparation according to one of the preceding claims, **characterised in that** it is a workable paste in the form of a water-in-silicone emulsion with a complex viscosity of 800 to 6,000 Pas and a zero viscosity of 200,000 to 1,200,000 Pas, preferably 400,000 and 900,000 Pas (shearing rate at zero viscosity 0.00005 s⁻¹; temperature 298.15 K).

40. A preparation according to one of the previous claims, **characterised in that** it is poured off into re-closable bottles, jars or tubes.

41. A preparation according to one of the previous claims, **characterised in that** it is poured off into a re-closable dispensing mechanism.

42. Method for the production of a preparation according to one of claims 1 to 41, wherein first the particulate phase is dispersed in the volatile silicone, separately therefrom the ester component is melted down and the hydrophobic ingredients are added, in a separate batch the aqueous phase is heated to the temperature of the ester component, and the hydrophilic ingredients are added, subsequently all three batches are brought together, homogenized and subsequently cooled and optionally as soon as the emulsion has reached approximately body temperature heat-sensitive constituents, such as fragrances, are added.

## Revendications

1. Préparation sous la forme d'une émulsion présentant une phase externe lipophile et une phase interne hydrophile, dans laquelle la phase externe contient au moins un ester polyvalent, au moins une silicone volatile et éventuellement d'autres additifs lipophiles usuels en cosmétique, et la phase interne contient un milieu aqueux et éventuellement des additifs hydrophiles usuels en cosmétique, dans laquelle la préparation contient en outre un émulsifiant et une phase solide, dans laquelle l'ester polyvalent est composé d'alcools au moins divalents avec respectivement au moins deux radicaux acide, ou d'acides au moins divalents et de respectivement au moins deux radicaux alcool, ou d'alcools polyvalents et d'acides polyvalents, dans laquelle la longueur de chaîne de radicaux provenant de l'alcool est en C₂ à C₆₀, et la longueur de chaîne des radicaux provenant de l'acide est en C₄ à C₆₀, dans laquelle l'ester présente un point de fusion dans la gamme de 40 à 200 °C.

2. Préparation, en particulier préparation cosmétique selon la revendication 1, **caractérisée en ce que** l'ester polyvalent présente d'autres groupes fonctionnels sélectionnés parmi des groupes hydroxyle, carboxyle, amino, amide d'acide ou ester.

3. Préparation selon la revendication 1 ou 2, **caractérisée en ce que** l'ester polyvalent est un composé de formule I: 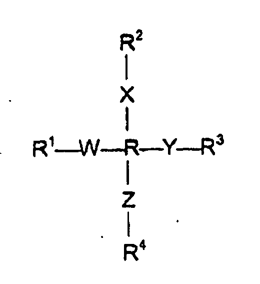 dans laquelle R est un radical hydrocarbure linéaire, ramifié ou cyclique comportant 1 à 8 atomes de carbone, W, X, Y et Z sont respectivement indépendamment les uns des autres -C(O)O-, -OC(O)-, -O-, -NR⁵₂ - NC(O)-, et chacun des radicaux R¹, R², R³, R⁴ et R⁵ représente indépendamment un radical hydrocarbure à chaîne longue linéaire ou ramifié.

4. Préparation selon la revendication 3, **caractérisée en ce que** l'ester polyvalent est un composé de formule I dans laquelle W, X, Y et Z représentent respectivement un groupe ester, R représente C et au moins trois des radicaux R¹, R², R³ et R⁴ représentent respectivement indépendamment un radical alkyle en C₆ à C₂₄ et le quatrième radical est H ou représente un radical alkyle en C₆ à C₂₄.

5. Préparation selon l'une des revendications précédentes, **caractérisée en ce que** dans le composant ester, la somme des atomes de carbone des radicaux alcool et acide carboxylique est dans une gamme de 35 à 150.

6. Préparation selon l'une des revendications précédentes, **caractérisée en ce que** l'ester polyvalent est un ester de pentaérythritol.

7. Préparation selon la revendication 6, **caractérisée en ce que** l'ester de pentaérythritol est le tétramyristate, le tristéarate, le tétrastéarate, le triisostéarate, le tétraisostéarate, le tribéhénate, le tétrabéhénate, le tétra-(éthylhexyldodécanoate), le tri-(12-hydroxy)-stéarate, le tétra-(12-hydroxy)stéarate, le triérucate, le tétraérucate, le tétramélissinate de pentaérythritol, ou un mélange de ceux-ci.

8. Préparation selon l'une des revendications précédentes, **caractérisée en ce qu'**elle contient exclusivement des ingrédients pouvant être dérivés de plantes et/ou minéraux et/ou synthétiques et est en ce sens totalement exempte de substances qui dérivent d'animaux.

9. Préparation selon l'une des revendications précédentes, **caractérisée en ce qu'**elle présente en outre une cire présentant un point de goutte entre 50 et 200 °C, de préférence entre 60 et 150 °C, de façon particulièrement préférée entre 75 et 120°C.

10. Préparation selon la revendication 7, **caractérisée en ce que** la cire présente tant au moins un radical alcool qu'au moins un radical acide carboxylique qui possède une fraction hydrocarbure linéaire ou ramifiée, saturée ou mono- ou polyinsaturée.

11. Préparation selon la revendication 10, **caractérisée en ce que** la cire est un mélange de cire de candellila et de cire de carnauba.

12. Préparation selon l'une des revendications précédentes, **caractérisée en ce que** l'ester polyvalent est contenu dans une gamme de 0,5 à 20 % en poids, de préférence dans une gamme de 2 à 12 % en poids.

13. Préparation selon l'une des revendications précédentes, **caractérisée en ce que** l'huile de silicone volatile est sélectionnée parmi l'héxaméthylcyclotrisiloxane, l'octaméthylcyclotétrasiloxane, le décaméthylcyclopentasiloxane, le dodécaméthylcyclohexasiloxane, l'hexaméthyldisiloxane, l'octaméthyltrisiloxane, le décaméthyltétrasiloxane, le dodécaméthylpentasiloxane ou des mélanges de ceux-ci.

14. Préparation selon la revendication 6, **caractérisée en ce qu'**est en outre contenue une huile de silicone non volatile dans une quantité inférieure à 5 % en poids.

15. Préparation, en particulier préparation cosmétique selon la revendication 14, **caractérisée en ce qu'**il s'agit concernant l'huile de silicone d'une huile de silicone non volatile ou d'un mélange d'huiles de silicones non volatiles.

16. Préparation selon la revendication 15, **caractérisée en ce que** la silicone non volatile est sélectionnée parmi des diméthylpolysiloxanes présentant différentes longueurs de chaîne et une viscosité différente ou des huiles de silicone arylées, comme des phényldiméthicones, des phényltriméthicones, des diphényldiméthicones ou des mélanges de celles-ci.

17. Préparation selon l'une des revendications précédentes, **caractérisée en ce que** l'émulsifiant est un émulsifiant eau/huile ou un mélange d'émulsifiant eau/huile et eau/silicone.

18. Préparation selon la revendication 17, **caractérisée en ce que** l'émulsifiant est un émulsifiant eau/huile non ionogène.

19. Préparation selon la revendication 18, **caractérisée en ce que** l'émulsifiant eau/huile non ionogène est sélectionné parmi le sesquioléate de sorbitane, le laurate de sorbitane, le stérol de soja, le soja-stérol de PEG-5, l'isostéarate de polyglycéryle 4, l'isostéarate de polyglycéryle-2-PEG-4, le sesquiisostéarate de polyglycéryle-2, la cétyl-PEG/PPG-diméthicone, le phosphate de trioléyle, le phosphate de trioleth-8, le phosphate de trilaureth-4 ou des mélanges de ceux-ci.

20. Préparation selon l'une des revendications précédentes, **caractérisée en ce qu'**elle contient un agent de stabilisation.

21. Préparation selon la revendication 20, **caractérisé en ce que** l'agent de stabilisation est un sel inorganique légèrement soluble dans l'eau.

22. Préparation selon la revendication 21, **caractérisé en ce que** le sel inorganique légèrement soluble dans l'eau est sélectionné parmi le chlorure de sodium, le chlorure de potassium, le sulfate de sodium, le sulfate de magnésium ou des mélanges de ceux-ci.

23. Préparation selon l'une des revendications 19 à 21, **caractérisée en ce que** l'agent de stabilisation est contenu dans la phase aqueuse dans une quantité de 0,05 à 3 % en poids, de préférence dans une quantité de 0,3 à 2 % en poids.

24. Préparation selon l'une des revendications précédentes, **caractérisée en ce qu'**un humectant est en outre contenu dans la phase aqueuse.

25. Préparation selon la revendication 24, **caractérisée en ce que** l'humectant est sélectionné parmi le propylèneglycol, le dipropylèneglycol, le tripropylèneglycol, le butylèneglycol, la glycérine, la diglycérine, la triglycérine, le sorbitol, le mannitol, le xylitol, le glucose, le fructose, le saccharose, le carbamide (urée), l'acide lactique, l'acide citrique, l'acide pyrrolidonecarboxylique (PCA) ou des sels des acides cités, ou des mélanges des substances citées.

26. Préparation selon l'une des revendications 24 ou 25, **caractérisée en ce que** l'humectant est contenu dans la phase aqueuse dans une quantité de 0,1 à 5 % en poids, de préférence dans une quantité de 1 à 3 % en poids.

27. Préparation selon l'une des revendications précédentes, **caractérisée en ce que** la phase solide présente des matières de charge, des substances d'effet et/ou des pigments inorganiques et/ou organiques ou leurs mélanges.

28. Préparation selon la revendication 27, **caractérisée en ce que** la matière de charge est sélectionnée parmi le talc, le kaolin, l'amidon, l'amidon modifié, la poudre de polytétrafluoroéthylène, la poudre de nylon, le nitrure de bore, le stéarate de Mg, le stéarate de Ca, le stéarate de Sr, le stéarate de Zn ou leurs mélanges.

29. Préparation selon la revendication 27 ou 28, **caractérisée en ce que** la phase solide est contenue dans des fractions en quantité dans une gamme de 0 à 40 % en poids, de préférence de 5 à 30 % en poids, de façon particulièrement préférée de 8 à 20 % en poids.

30. Préparation selon la revendication 27, **caractérisée en ce que** le pigment inorganique est un nanopigment présentant une taille de particules de 5 à 50 nm, sélectionné parmi le dioxyde de titane, l'oxyde de zinc, l'oxyde de zirconium, l'oxyde de cérium, l'oxyde d'aluminium, le dioxyde de silicium ou leurs mélanges.

31. Préparation selon la revendication 30, **caractérisée en ce que** le nanopigment est contenu dans une quantité de 2 à 20 % en poids, de préférence dans une quantité de 5 à 10 % en poids.

32. Préparation selon l'une des revendications 30 à 31, **caractérisée en ce que** le nanopigment est combiné à des substances filtrant la lumière UVA et UVB solubles dans l'huile.

33. Préparation selon la revendication 32, **caractérisée en ce que** les substances filtrant la lumière UVA et UVB solubles dans l'huile sont le 4-méthylbenzylidène-camphre et le p-méthoxycinnamate d'isoamyle.

34. Préparation selon la revendication 27, **caractérisée en ce que** le pigment inorganique est le dioxyde de titane, l'oxyde de zinc, l'oxyde de fer, l'oxyde de chrome, l'oxyde de chrome hydraté, l'ultramarine, le bleu de Berlin (bleu ferrique), le mica, un mica enrobé de dioxyde de titane, un mica enrobé de dioxyde de titane et d'oxydes métalliques, l'oxychlorure de bismuth, l'oxychlorure de bismuth enrobé, une poudre métallique d'aluminium en paillettes, le laiton, le bronze, le cuivre, l'argent, l'or, ou des mélanges de ceux-ci.

35. Préparation selon la revendication 27, **caractérisée en ce que** le pigment organique est présent sous la forme de formations de colorants organiques avec de l'aluminium, du baryum, du calcium, du strontium, du zirconium et leurs mélanges.

36. Préparation, en particulier préparation cosmétique selon l'une des revendications précédentes, **caractérisée en ce qu'**elle est appropriée dans le secteur de la cosmétique de maquillage pour soigner, colorer et embellir la peau, les lèvres et les paupières.

37. Préparation selon l'une des revendications précédentes, **caractérisée en ce qu'**elle est un rouge à lèvres, un rouge à joues, du maquillage, des ombres à paupières, un produit correcteur ou un cache-cernes.

38. Préparation selon l'une des revendications précédentes, **caractérisée en ce qu'**elle est un moyen pour stabiliser le baume à lèvres ou le rouge à lèvres, une base de soin un agent de soin de la peau ou un agent de protection solaire.

39. Préparation selon l'une des revendications précédentes, **caractérisée en ce qu'**elle est une pâte souple sous la forme d'une émulsion eau-dans-silicone, présentant une viscosité complexe de 800 à 6 000 Pas et une viscosité à cisaillement nul de 200 000 à 1 200 000 Pas, de préférence de 400 000 à 900 000 Pas (vitesse de cisaillement pour la viscosité à cisaillement nul 0,00005 s⁻¹, température 298,15 K).

40. Préparation selon l'une des revendications précédentes, **caractérisée en ce que**lle est placée dans des flacons ou bouteilles, des pots et des tubes refermables.

41. Préparation selon l'une des revendications précédentes, **caractérisée en ce qu'**elle est placée dans un mécanisme distributeur refermable.

42. Procédé de fabrication d'une préparation selon l'une des revendication 1 à 41, dans lequel la phase particulaire est d'abord dispersée dans la silicone volatile, séparément les composants ester sont fondus et les ingrédients hydrophobes sont ajoutés, dans une préparation séparée la phase aqueuse est chauffée à la température des composants ester et les ingrédients hydrophiles sont ajoutés, puis toutes les trois préparations sont réunies, homogénéisées puis refroidies, et éventuellement, dès que l'émulsion a atteint environ la température corporelle, les composants sensibles à la température, comme les substances odorantes, sont ajoutés.
